(19) Europäisches Patentamt
European Patent Office
Office européen des brevets

(11) EP 0 813 412 B1

(12) # FASCICULE DE BREVET EUROPEEN

(45) Date de publication et mention de la délivrance du brevet:
**29.12.1999 Bulletin 1999/52**

(21) Numéro de dépôt: **96933493.7**

(22) Date de dépôt: **04.10.1996**

(51) Int. Cl.$^6$: **A61K 31/565**, A61K 9/00

(86) Numéro de dépôt international:
**PCT/FR96/01556**

(87) Numéro de publication internationale:
**WO 97/12618 (10.04.1997 Gazette 1997/16)**

(54) **GEL POUR HORMONOTHERAPIE LOCALE DE LA SECHERESSE VAGINALE**
GEL ZUR LOKALEN HORMONTHERAPIE DER VAGINALEN TROCKENHEIT
GEL FOR THE LOCAL HORMONOTHERAPY OF VAGINAL DRYNESS

(84) Etats contractants désignés:
**AT BE CH DE DK ES FI FR GB GR IE IT LI LU MC NL PT SE**

(30) Priorité: **05.10.1995 FR 9511731**

(43) Date de publication de la demande:
**29.12.1997 Bulletin 1997/52**

(73) Titulaire:
**LABORATOIRE INNOTHERA Société Anonyme**
**94111 Arcueil (FR)**

(72) Inventeurs:
- **MEIGNANT, Catherine**
  **F-75010 Paris (FR)**
- **VIEILLARD-BARON, Corinne**
  **F-75015 Paris (FR)**

(74) Mandataire:
**Dupuis-Latour, Dominique et al**
**Avocat à la Cour,**
**Cabinet Bardehle, Pagenberg & Partner,**
**14 boulevard Malesherbes**
**75008 Paris (FR)**

(56) Documents cités:
**EP-A- 0 587 047** **FR-A- 2 355 510**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 813 412 B1

Printed by Xerox (UK) Business Services
2.16.7/3.6

## Description

L'invention concerne une composition pharmaceutique pour le traitement local, essentiellement non systémique, de la sécheresse vaginale.

On connaît les inconvénients de la sécheresse vaginale, notamment chez la femme ménopausée : dyspareunie, atrophie urogénitale pouvant entraîner des perturbations de la fonction urinaire, risques d'infection en raison d'une flore insuffisamment développée.

L'un des buts de l'invention est de proposer une composition convenant à un traitement essentiellement non systémique, qui se distingue donc des traitements d'hormonothérapie substitutive, où l'hormone peut être administrée *per os*, par voie transcutanée ou par administration intravaginale.

La composition de l'invention se distingue en particulier des compositions d'hormonothérapie substitutive administrées par voie vaginale, par exemple sous forme de crèmes, qui sont fortement dosées en estrogènes.

Le FR-A-2 355 510 décrit ainsi un gel adapté à une application notamment vaginale, et pouvant contenir entre autres principes actifs un agent hormonal tel que l'estradiol. Une telle formulation permet de réaliser une hormonothérapie par administration intravaginale, mais il s'agit simplement dans ce cas de tirer parti du meilleur passage systémique de la voie vaginale par rapport à la voie orale, compte tenu notamment d'une absence de métabolisation des estrogènes lorsque l'on utilise cette voie. Ce document suggère en outre le choix d'un gel de viscosité faible ou moyenne, à savoir de 5000 à 100 000 centipoises (g/m.s ou mPa.s) pour accélérer l'absorption du principe actif par l'organisme.

L'invention cherche au contraire à réaliser un traitement local, avec passage systémique minime ou nul, par apport direct d'un estrogène naturel, et plus particulièrement du 17β-estradiol, permettant de soulager les troubles locaux en évitant les effets secondaires systémiques susceptibles d'apparaître chez certaines patientes, notamment l'apparition d'une hyperplasie endométriale.

On a déjà proposé des traitements locaux de ce type, par exemple sous forme d'un anneau vaginal en forme de tore enfermant un estrogène qui diffuse au travers de la membrane poreuse de l'anneau, permettant ainsi une libération continue sur une longue durée.

Ces anneaux présentent cependant l'inconvénient, comme tout dispositif intravaginal, d'une part d'impliquer la présence dans l'organisme d'un corps étranger non dégradable et d'autre part de nécessiter des manipulations pour sa pose et son retrait. En outre, il ne procure aucune lubrification de la muqueuse et il peut être nécessaire d'utiliser en complément des agents lubrifiants neutres (ne contenant pas de principe actif).

Il a été également proposé pour ce traitement local une forme galénique comportant du 17β-estradiol sous forme de comprimés vaginaux administrés quotidiennement. Ces comprimés sont des comprimés matriciels comprenant un excipient tel qu'un polymère cellulosique absorbant les traces d'humidité vaginale résiduelle de manière à imprégner la matrice contenant le principe actif et à libérer celui-ci de façon progressive.

Toutefois, du fait que ces comprimés ne sont pas en contact direct avec l'ensemble de la région à traiter et que le principe actif doit s'étendre progressivement sur celle-ci, leur dosage doit être relativement élevé pour procurer les résultats voulus, typiquement un dosage de 25 μg de 17β-estradiol par comprimé (un comprimé correspondant à une dose quotidienne) pour permettre l'amélioration cytologique, histologique et clinique recherchée de la muqueuse vaginale. Du fait de cette dose relativement élevée, on note d'après les études cliniques une prolifération de l'endomètre chez certaines patientes, signe d'un passage systémique du 17β-estradiol : voir notamment C. Felding et coll., Preoperative Treatment with OEstradiol in Women Scheduled for Vaginal Operation for Genital Prolapse. A Randomised, Double-Blind Trial, *Maturitas,* 1992, *15,* 241-249.

De plus, ces comprimés ne procurent aucune lubrification de la muqueuse et il peut être nécessaire d'utiliser en complément, ici encore, des agents lubrifiants neutres.

L'un des buts de la présente invention est de proposer, dans l'indication spécifique précitée - à savoir le traitement de la sécheresse vaginale - une composition élaborée avec une forme galénique particulière permettant de réduire le dosage du 17β-estradiol de manière à éviter le passage systémique malgré l'extrême sensibilité des muqueuses vaginales aux estrogènes, mais tout en procurant une efficacité trophique satisfaisante.

En d'autres termes, il s'agit de trouver une forme galénique particulière qui, par synergie entre le vecteur et le principe actif, potentialise l'effet de ce dernier et permette donc de réduire très fortement le dosage en 17β-estradiol par rapport à l'art antérieur (on verra plus loin que cette réduction du dosage est de l'ordre de 40 à 90 %).

Essentiellement, la composition de l'invention est caractérisée par la combinaison synergique d'un estrogène naturel choisi parmi le 17β-estradiol et ses sels et d'un gel biodégradable, hydrophile, lubrifiant et bioadhésif, la teneur en estrogène par dose unitaire équivalente appliquée étant d'au plus 15 μg, de préférence comprise entre 2,5 μg et 15 μg, de préférence moins de 10 μg, de 17β-estradiol, cette spécialité étant destinée à être appliquée sur la muqueuse vulvo-vaginale.

Le caractère hydrophile de l'excipient permet notamment une diffusion passive directe du principe actif entre l'excipient et la muqueuse vaginale avec laquelle celui-ci est en contact. Le caractère bioadhésif (plus précisément,

mucoadhésif) permet de rendre le gel collant sur la muqueuse, avec un faible écoulement, procurant ainsi un maintien de longue durée.

Ce maintien de longue durée permet en particulier d'espacer les applications, qui peuvent n'être que quotidiennes, ou encore moins fréquentes (notamment en phase d'entretien).

Le caractère lubrifiant du gel tire avantageusement parti du maintien prolongé de celui-ci sur la muqueuse, et évite d'avoir recours à un lubrifiant de complément.

Selon un certain nombre de caractéristiques avantageuses :

- la viscosité du gel est comprise entre 200 000 et 600 000 centipoises (g/m.s ou mPa.s) ;
- l'estrogène, avantageusement micronisé, est présent dans le gel soit sous forme libre soit sous forme vectorisée, notamment par encapsulation dans des vecteurs de type nanoparticules tels que des biovecteurs supramoléculaires ;
- le gel comprend un composant hydrophile bioadhésif gélifiant choisi parmi les acides carboxyvinyliques, l'hydroxypropylcellulose, la carboxyméthylcellulose, la gélatine, la gomme xanthane, la gomme Guar, le silicate d'aluminium et leurs mélanges ;
- le gel comprend un composant hydrophile émollient choisi parmi la glycérine, les propylène glycols, les polyoxyéthylène glycols et leurs mélanges ;
- le gel comprend de l'acide carboxyvinylique, de la glycérine, une substance d'ajustement du pH, un adjuvant conservateur et un excipient aqueux ;
- la composition de la spécialité est: 17β-estradiol micronisé libre ou vectorisé, 0,0625 mg à 0,375 mg (correspondant à une dose unitaire de 2,5 à 15 μg pour 4 g de gel formulé) ; acide carboxyvinylique, 1 à 3 g ; glycérine, 5 à 15 g ; parahydroxybenzoate de méthyle, 0,025 g ; parahydroxybenzoate de propyle, 0,025 g ; hydroxyde de sodium q.s. pH 4 ± 0,5 ; eau purifiée q.s. 100 g.

On va maintenant décrire plus en détail les différents aspects de la présente invention, en donnant ensuite divers exemples de formulations.

*Choix du principe actif*

On choisit pour la composition de l'invention un estrogène choisi parmi le 17β-estradiol, ses sels et ses dérivés.

Ce groupe comprend la famille de composés dont la structure chimique répond à la formule générale :

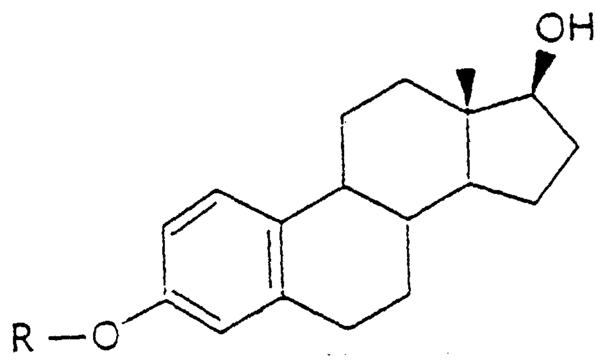

Lorsque R = H , le composé est le 17β-estradiol, qui est l'hormone naturelle produite physiologiquement par les ovaires des femmes fertiles et dont la carence est responsable des troubles fonctionnels ressentis chez la patiente ménopausée.

Le 17β-estradiol est un agoniste estrogénique physiologique. Son rôle trophique sur la muqueuse vulvo-vaginale est reconnu et a été largement décrit, de même que la réversibilité des troubles histologiques fonctionnels et cliniques par administration de 17β-estradiol. L'estradiol et ses dérivés (sels) diminuent le pH vaginal et augmentent la différence de potentiel transvaginal, la quantité de sécrétions vaginales et le débit sanguin local.

Chez la femme, des récepteurs à haute affinité pour l'estradiol ont été décelés au niveau de l'épithélium vaginal. Ceux-ci présentent pour l'estradiol radiomarqué une affinité voisine de celle calculée pour les récepteurs du myomètre mais leur nombre paraît plus faible. Ces ré-cepteurs se caractérisent par une affinité décroissante pour les composés suivants : 17β-estradiol > estriol > estrone.

Mais alors que le 17β-estradiol se révèle être un agoniste pur, l'estriol, son métabolite naturel, se caractérise par des propriétés d'agoniste partiel, voire d'antagoniste. Un effet antagoniste pourrait se manifester vis à vis de l'estrogène naturel.

Un traitement par le 17β-estracliol présente donc l'avantage de l'additivité des effets en présence de l'estradiol endogène, alors qu'un traitement par l'estriol présente l'inconvénient d'un effet antagoniste vis-à-vis de l'estradiol endogène. De plus, lestriol, du fait de son activité intrinsèque plus faible que celle du 17β-estradiol, se révèle moins actif que celui-ci (certains auteurs expliquent ce phénomène par une plus grande vitesse de dissociation de ses récepteurs nucléaires).

Comme l'activité présentée par un agoniste partiel est plus dépendante du nombre de récepteurs que celle de l'agoniste complet, la différence d'activité entre ces deux composés estrogéniques est d'autant plus marquée que le taux de récepteurs tissulaires vaginaux paraît plus faible qu'au niveau du myomètre.

En conclusion, vu la haute affinité du 17β-estradiol pour les récepteurs estrogéniques vaginaux et surtout son profil d'activité en tant qu'agoniste complet, cet estrogène naturel constitue un meilleur choix que l'estriol pour obtenir un effet trophique local.

Les mêmes remarques s'appliquent à l'estrone, qui est un précurseur de l'estriol, ainsi qu'aux dérivés de synthèse de l'estradiol tels que notamment le diétheroxyde d'estradiol (promestriène DCI).

*Choix de la forme gel*

L'utilisation d'un gel comme forme galénique présente de nombreux avantages à l'égard du but recherché.

En premier lieu, il assure un contact direct et prolongé sur toute l'étendue de la muqueuse à traiter et assure ainsi une action topique optimale sur celle-ci.

En second lieu, on peut délibérément donner au gel une viscosité élevée pour le rendre collant sur la muqueuse afin de permettre un maintien prolongé et un faible écoulement (phénomène de "bioadhésion" ou "mucoadhésion"). Quantitativement, ceci correspond à une viscosité comprise entre 200 000 et 600 000 centipoises (g/m.s ou mPa.s).

Cette caractéristique est particulièrement avantageuse dans la mesure où elle permet d'espacer les applications, typiquement jusqu'à une application quotidienne, voire encore moins fréquente.

Dans les exemples de formulations donnés plus bas, on a ainsi constaté que le gel était toujours présent même vingt-quatre heures après l'examen, et en une quantité encore notable, au point de gêner l'examen.

Un autre avantage du choix d'un gel est qu'il présente un caractère lubrifiant rendant inutile une application additionnelle d'un agent lubrifiant neutre en cas de besoin. En outre, le gel se maintenant en place de façon prolongée comme indiqué plus haut, l'action lubrifiante est conservée pendant une longue durée.

*Choix du dosage*

Le dosage doit être choisi de manière à soulager les troubles locaux en évitant au maximum l'absorption transvaginale.

Pour atteindre ces objectifs, on choisit une dose de 10 μg de 17β-estradiol, correspondant à une dose unitaire de 4 g de gel formulé (application journalière unique, voire encore moins fréquente).

Lorsque le 17β-estradiol est présent à cette dose sous forme micronisée libre dans le gel, on n'observe qu'un léger passage systémique, sous forme d'un simple pic plasmatique au bout d'une heure environ après l'administration en dose unique ; la concentration plasmatique maximale du pic est du même ordre que le taux physiologique moyen observés la première semaine de la phase folliculaire chez la femme fertile, mais elle est bien évidemment très fugace, puisqu'en quatre à six heures les concentrations plasmatiques retrouvent des valeurs proches des valeurs basales.

Ce léger passage systémique est la plupart du temps parfaitement supporté par les patientes, sur lesquelles il n'est pas observé d'effets secondaires.

L'absence d'effets secondaires n'est toutefois pas systématique, et il peut être souhaitable de réduire encore plus le passage systémique, typiquement en imposant une concentration plasmatique qui reste toujours inférieure à 50 pg/ml pour toutes les patientes, afin d'éviter toutes les contre-indications des estrogènes.

Une première solution consiste à réduire la teneur en principe actif, typiquement à des doses de 5 μg ou même 2,5 μg par application unitaire. On n'observe dans ce cas aucun passage systémique significatif (au sens indiqué plus haut, c'est-à-dire que la concentration plasmatique de 17β-estradiol ne dépasse jamais 50 pg/ml).

Une autre possibilité consiste, en conservant le véhicule lubrifiant hydrophile, à vectoriser le principe actif au lieu de le mettre sous forme libre dans le véhicule.

On va expliquer ci-dessous l'intérêt de cette vectorisation et la manière dont on peut la réaliser.

*Vectorisation du 17β-estradiol*

Le but premier de la vectorisation du 17β-estradiol est principalement d'éliminer le passage systémique du principe actif par une libération plus progressive de ce dernier qui aura pour effet d'"étaler" le pic plasmatique en réduisant son amplitude maximale, qui pourra rester toujours inférieure à 50 pg/ml de concentration plasmatique.
Avantageusement, la vectorisation permettra également d'augmenter la durée d'action locale du principe actif.
Ces deux buts peuvent être atteints de la manière suivante.
Pour éviter tout passage systémique, la taille du vecteur doit être suffisamment élevée pour ne pas traverser l'épithélium vaginal. Une taille de l'ordre de 200 nm de diamètre répond à ce critère. Bien entendu, le vecteur doit être compatible avec le 17β-estradiol, permettre son relargage progressif, doit être compatible avec le mucus vaginal et parfaitement toléré.
Pour augmenter la durée d'action, on peut choisir un système de bioadhésion par interactions électrostatiques. En effet, dans des conditions normales, le mucus vaginal est de nature acide (pH de l'ordre de 4) tandis qu'à la ménopause ce pH a tendance à augmenter aux environs de 6. Il est donc intéressant d'apporter en périphérie du vecteur des charges positives qui peuvent ainsi interagir avec les charges négatives du mucus.
Il faut noter que les propriétés acides requises pour une interaction maximale entre le mucus et le vecteur sont réduites en période de ménopause (pH de l'ordre de 6), mais ces conditions de faible acidité peuvent être suffisantes pour une interaction efficace avec les vecteurs.
Un vecteur répondant à ces différentes conditions est par exemple constitué par des nanoparticules (c'est-à-dire des particules dont le diamètre est de l'ordre de quelques dizaines ou au plus quelques centaines de nanomètres) tels que les "biovecteurs supramoléculaires" (BVSM) décrits dans le WO-A-89/11271 (Centre national de la recherche scientifique) et produits par la société Biovector Therapeutics SA.
Ces BVSM, qui sont des vecteurs en eux-mêmes connus, comprennent un noyau hydrophile non liquide, une enveloppe interne de nature lipidique liée au noyau par des liaisons covalentes et une enveloppe externe amphiphile liée à l'enveloppe interne lipidique par des interactions hydrophobes.
Ces vecteurs peuvent être chargés en principe actif, ici par le 17β-estradiol (qui est lipophile) encapsulé dans le vecteur, l'ensemble constituant alors un transporteur de principe actif biomimétique des systèmes de transport endogènes telles que les lipoprotéines.

*Exemples de formulation*

On va maintenant donner des exemples de formulation du gel selon l'invention.
Une formule typique peut être la suivante :

| | |
|---|---|
| 17β-estradiol micronisé libre ou vectorisé correspondant à une dose unitaire de 2,5 à 15 μg pour 4 g de gel formulé | 0,0625 mg à 0,375 mg |
| Acide carboxyvinylique (Carbopol® 974 P) | 2 g |
| Glycérine | 10 g |
| Parahydroxybenzoate de méthyle | 0,025 g |
| Parahydroxybenzoate de propyle | 0,025 g |
| Hydroxyde de sodium | q.s. pH 4 ± 0,5 |
| Eau purifiée | q.s. 100 g |

On notera la très faible concentration finale en principe actif, qui est de $0{,}625.10^{-6}$ à $3{,}75.10^{-6}$ pour la plage de doses unitaires indiquée ci-dessus, en particulier de $2{,}5.10^{-6}$ (0,00025 %) dans le cas correspondant aux essais cliniques dont on rendra compte plus bas.
Diverses variations de dosage des différents excipients peuvent être envisagées. Ainsi, la dose de Carbopol® 974 P peut être comprise entre 1 et 3 g, et celle de glycérine entre 5 et 15 g.
Il est également possible de modifier la composition des excipients.
Ainsi, le Carbopol® 974 P peut être remplacé par d'autres composants hydrophiles gélifiants bioadhésifs tels que :

— hydroxypropylcellulose, entre 1 et 3 g,
— carboxyméthylcellulose, entre 1 et 5 g,
— gélatine, entre 1 et 5 g,
— gomme xanthane, entre 1 et 2 g,
— gomme Guar, entre 1 et 2 g,
— silicate d'aluminium, entre 1 et 2 g, ou
— un mélange de deux ou plusieurs des composants précédents.

La glycérine peut être remplacée par d'autres composants hydrophiles émollients tels que :

— propylène glycol, entre 5 et 8 g,
— polyoxyéthylène glycol (PEG), entre 5 et 15 g, ou
— un mélange des composants précédents.

*Essais cliniques*

Les résultats obtenus mettent en évidence les éléments suivants.

Après administration répétée de 10 µg de 17β-estradiol au niveau vaginal pendant 13 jours, la tolérance clinique a été excellente pour treize sujets sur seize. Trois sujets ont signalé des effets indésirables (céphalées, épisodes de vomissements et bouffées de chaleur) qui peuvent être reliés au produit étudié compte tenu du passage systémique mesuré dans cet essai. Ces événements d'intensité mineure n'ont pas empêché la poursuite de l'étude. La tolérance biologique a été excellente. Aucune anomalie ayant une signification clinique n'a été rapportée.

Du point de vue pharmacodynamique, il n'a pas été observé de changement significatif au niveau des taux sanguins de FSH, LH et SHBG (*Sexual Hormonal Binding Globulin*) durant toute la période d'administration.

L'application répétée de 10 µg de 17β-estradiol au niveau vaginal pendant 13 jours provoque une régénération statistiquement significative des trois types cellulaires de la muqueuse vaginale dès le Jour 7. Cette régénération statistiquement significative existe encore entre le Jour 7 et le Jour 14 pour les cellules vaginales superficielles qui étaient totalement absentes en début de traitement.

Cette régénération de la muqueuse vaginale s'accompagne d'une diminution du pH vaginal et d'une amélioration de la symptomatologie fonctionnelle (sécheresse vaginale, dyspareunie, prurit, leucorrhées desquamantes récidivantes et dysurie) dès le septième jour d'application et persistantes sans changement significatif jusqu'au quatorzième jour d'application.

Du point de vue pharmacocinétique, les concentrations plasmatiques de 17β-estradiol augmentent dès la première application du gel vaginal. A l'état d'équilibre, le pic plasmatique d'estradiol tend à être plus faible et retardé, la surface sous la courbe étant comparable à celle obtenue après la première application.

L'augmentation des concentrations d'estrone par rapport aux taux de base est faible comparée à celle des concentrations de 17β-estradiol, que l'on considère la première ou la dernière application du gel de 17β-estradiol. Ceci traduit d'une part l'absence d'effet de premier passage pour le 17β-estradiol et explique d'autre part la valeur obtenue pour le ratio estradiol/estrone (1,83 ± 0,90), qui est comparable au ratio rapporté chez la femme préménopausée en phase folliculaire tardive.

On notera que ces essais cliniques, qui sont déjà particulièrement concluants, ont été menés avec la dose maximale prévue, et pour du 17β-estradiol micronisé non vectorisé.

Les effets secondaires apparaissant chez quelques sujets peuvent être réduits en diminuant la dose (par exemple des doses unitaires de 5 µg, ou même 2,5 µg, d'estradiol libre), et/ou en vectorisant le 17β-estradiol pour étaler dans le temps le pic de concentration plasmatique et donc abaisser son niveau maximal au-dessous du niveau de 50 pg/ml susceptible d'induire des effets secondaires.

**Revendications**

1. Une composition pharmaceutique pour utilisation à titre de médicament en application locale pour le traitement essentiellement non systémique de la sécheresse vaginale, notamment chez la femme ménopausée, caractérisée par la combinaison synergique d'un estrogène naturel choisi parmi le 17β-estradiol et ses sels et d'un gel biodégradable, hydrophile, lubrifiant et bioadhésif,

   la teneur en estrogène par dose unitaire appliquée étant d'au plus 15 µg de 17β-estradiol, de préférence comprise entre 2,5 µg et 15 µg, de préférence moins de 10 µg,
   cette composition étant destinée à être appliquée sur la muqueuse vulvo-vaginale.

2. La composition de la revendication 1, dans laquelle la viscosité du gel est comprise entre 200 000 et 600 000 centipoises (g/m.s ou mPa.s).

3. La composition de la revendication 1, dans laquelle l'estrogène est présent sous forme libre, avantageusement micronisée, dans le gel.

4. La composition de la revendication 1, dans laquelle l'estrogène est présent sous forme vectorisée, avantageusement micronisée, dans le gel.

5. La composition de la revendication 4, dans laquelle l'estrogène est vectorisé par encapsulation dans des vecteurs de type nanoparticules.

6. La composition de la revendication 5, dans laquelle l'estrogène est vectorisé par encapsulation dans des vecteurs particulaires de type biovecteurs supramoléculaires.

7. La composition de la revendication 1, dans laquelle le gel comprend un composant hydrophile bioadhésif gélifiant choisi parmi les acides carboxyvinyliques, l'hydroxypropylcellulose, la carboxyméthylcellulose, la gélatine, la gomme xanthane, la gomme Guar, le silicate d'aluminium et leurs mélanges.

8. La composition de la revendication 1, dans laquelle le gel comprend un composant hydrophile émollient choisi parmi la glycérine, les propylène glycols, les polyoxyéthylène glycols et leurs mélanges.

9. La composition de la revendication 1, dans laquelle le gel comprend de l'acide carboxyvinylique, de la glycérine, une substance d'ajustement du pH, un adjuvant conservateur et un excipient aqueux.

10. La composition de la revendication 9, contenant :

| | |
|---|---|
| 17β-estradiol micronisé libre ou vectorisé correspondant à une dose unitaire de 2,5 à 15 µg pour 4 g de gel formulé | 0,0625 mg à 0,375 mg |
| Acide carboxyvinylique (Carbopol® 974 P) | 2 g |
| Glycérine | 10 g |
| Parahydroxybenzoate de méthyle | 0,025 g |
| Parahydroxybenzoate de propyle | 0,025 g |
| Hydroxyde de sodium | q.s. pH 4 ± 0,5 |
| Eau purifiée | q.s. 100 g |

## Claims

1. A pharmaceutical composition to be used as a medicament for local application for essentially non systemic treatment of vaginal dryness, in particular in the menopausal woman,
characterized by a synergistic combination of a natural estrogen selected from 17β-estradiol and its salts and a biodegradable hydrophilic lubricating and bioadhesive gel,

 the amount of estrogen by unit dose applied being at most 15 µg of 17β-estradiol, preferably in the range from 2.5 µg to 15 µg,
 said composition being intended for application to the vulvo-vaginal mucous membrane.

2. The composition of claim 1, in which the viscosity of the gel is in the range 200000 centipoises to 600000 centipoises (g/m.s or mPa/s).

3. The composition of claim 1, in which the estrogen is present in the gel in its free form, advantageously micronized.

4. The composition of claim 1, in which the estrogen is present in the gel in its vectorized form, advantageously micronized.

5. The composition of claim 4, in which the estrogen is vectorized by encapsulation in nanoparticle type vectors.

6. The composition of claim 5, in which the estrogen is vectorized by encapsulation in particulate supramolecular biovector type vectors.

7. The composition of claim 1, in which the gel comprises a hydrophilic bioadhesive gel-forming component selected from carboxyvinylic acids, hydroxypropylcellulose, carboxymethylcellulose, gelatin, xanthane gum, guar gum, aluminum silicate and mixtures thereof.

8. The composition of claim 1, in which the gel comprises a hydrophilic emollient component selected from glycerine, propylene glycols, polyoxyethylene glycols and mixtures thereof.

9. The composition of claim 1, in which the gel comprises carboxyvinylic acid, glycerine, a pH adjusting compound, a preservative and an aqueous excipient.

10. The composition of claim 9, including:

| | |
|---|---|
| Free or vectorized micronized 17β-estradiol corresponding to a unit dose of 2.5 μg to 15 μg per 4 g of formulated gel | 0.0625 mg to 0.375 mg |
| Carboxyvinylic acid (Carbopol® 974P) | 2 g |
| Glycerine | 10 g |
| Methyl parahydroxybenzoate | 0.025 g |
| Propyl parahydroxybenzoate | 0.025 g |
| Sodium hydroxide | q.s. pH 4 ± 0.5 |
| Purified water | g.s. 100 g |

**Patentansprüche**

1. Pharmazeutische Zusammensetzung zur Verwendung bei der Herstellung eines Medikaments zur lokalen Anwendung bei der Behandlung von im wesentlichen nicht systemischer Trockenheit der Vagina, insbesondere bei Frauen in der Menopause, gekennzeichnet durch die synergetische Kombination eines natürlichen Estrogens ausgewählt aus 17β-Estradiol und seinen Salzen und einem biologisch abbaubaren, hydrophilen und bioadhäsiven Gleit-Gel,

   wobei der Gehalt an Estrogen in der angewendeten Dosiseinheit höchstens 15 μg 17β-Estradiol, vorzugsweise zwischen 2,5 μg und 15 μg, vorzugsweise weniger als 10 μg beträgt,
   wobei die Zusammensetzung dazu bestimmt ist, auf die vulvo-vaginale Schleimhaut aufgetragen zu werden.

2. Zusammensetzung nach Anspruch 1, in welcher die Viskosität des Gels zwischen 200 000 und 600 000 Zentipoise (g/m.s oder mP.s) beträgt.

3. Zusammensetzung nach Anspruch 1, in welcher das Estrogen in freier Form, vorteilhafterweise mikronisiert, in dem Gel vorliegt.

4. Zusammensetzung nach Anspruch 1, in welcher das Estrogen in vektorisierter Form, vorteilhafterweise mikronisiert, in dem Gel vorliegt.

5. Zusammensetzung nach Anspruch 4, in welcher das Estrogen durch Einkapselung in Vektoren vom Typ der Nanopartikel vektorisiert ist.

**6.** Zusammensetzung nach Anspruch 5, in welcher das Estrogen durch Einkapselung in Partikelvektoren vom Typ der supramolekularen Biovektoren vektorisiert ist.

**7.** Zusammensetzung nach Anspruch 1, in welcher das Gel einen hydrophilen, bioadhäsiven Gel-Bestandteil umfaßt, ausgewählt aus Carboxyvinylsäuren, Hydroxypropylcellulose, Carboxymethylcellulose, Gelatine, Xanthangummi, Guargummi, Aluminiumsilikat und Gemischen davon.

**8.** Zusammensetzung nach Anspruch 1, in welcher das Gel einen hydrophilen weichmachenden Bestandteil umfaßt, ausgewählt aus Glycerin, Propylenglykolen, Polyoxyethylenglykolen und Gemischen davon.

**9.** Zusammensetzung nach Anspruch 1, in welcher das Gel Carboxyvinylsäure, Glycerin, eine den pH einstellende Substanz, ein konservierendes Adjuvans und einen wässrigen Excipienten umfaßt.

**10.** Zusammensetzung nach Anspruch 9, enthaltend:

| | |
|---|---|
| mikronisiertes 17β-Estradiol, frei oder vektorisiert entsprechend einer Dosiseinheit von 2,5 µg bis 15 µg auf 4 g hergestelltes Gel | 0,0625 mg bis 0,375 mg |
| Carboxyvinylsäure (Carbopol®974P) | 2 g |
| Glycerin | 10 g |
| Methyl-parahydroxybenzoat | 0,025 g |
| Propyl-parahydroxybenzoat | 0,025 g |
| Natriumhydroxid | q.s. bzw. höherer Qualität, pH 4 ± 0,5 |
| gereinigtes Wasser | q.s. bzw. höherer Qualität, 100 g |